# EUROPEAN PATENT APPLICATION

(11) **EP 1 300 146 A1**
(43) Date of publication of application: **09.04.2003**
(21) Application number: 01123700.5
(22) Date of filing: 03.10.2001
(51) Int. Cl.: A61K 31/519, A61P 35/00

(54) **Pharmaceutical composition for the treatment of animal mammary tumors**

(71) Applicant: Boehringer Ingelheim International GmbH, 55216 Ingelheim (DE)
(72) Inventor: Hilberg, Frank Dr., 1050 Wien (AT); Brandstetter, Iris, 1050 Wien (AT); Van Meel, Jacobus Dr., 2340 Mödling (AT); Bette, Peter Dr., 88437 Maselheim (DE); Kleemann, Rainer Dr., 55218 Ingelheim (DE)
(74) Representative: Laudien, Dieter, Dr.

(57) **Abstract**

Pharmaceutical composition for the treatment of mammary tumors of a non-human mammal, which is associated with an aberrant activity of one or more tyrosine kinase receptors. The composition contains, as the active ingredient, one or more substances that inhibit the aberrant activity of the tyrosine kinase receptor(s) of said animal, in particular the Epidermal Growth Factor Receptor (EGFR). The pharmaceutical composition is particularly useful for the treatment of dogs.

SEQUENCE LISTING

<110> Boehringer Ingelheim International GmbH

<120> Pharmaceutical composition for the treatment of animal
      mammary tumors

<130> Case 12_221

<140>
<141>

<160> 22

<170> PatentIn Ver. 2.1

<210> 1
<211> 1935
<212> DNA
<213> Canis familiaris

<220>
<221> CDS
<222> (1)..(1935)

<400> 1
cct cag gcc atg aac atc acc tgc aca gga cgg ggg ccg gac agc tgc    48
Pro Gln Ala Met Asn Ile Thr Cys Thr Gly Arg Gly Pro Asp Ser Cys
  1               5                  10                  15

atc aag tgc gcc cac tac atc gat ggc cct cac tgc gtc aag acc tgc    96
Ile Lys Cys Ala His Tyr Ile Asp Gly Pro His Cys Val Lys Thr Cys
             20                  25                  30

ccg gct ggc atc atg gga gaa aac aac acc ctg gtc tgg aag ttt tcg    144
Pro Ala Gly Ile Met Gly Glu Asn Asn Thr Leu Val Trp Lys Phe Ser
         35                  40                  45

gat ggc agc cgc atg tgc cac ctg tgc cat cca aac tgc acc tat ggc    192
Asp Gly Ser Arg Met Cys His Leu Cys His Pro Asn Cys Thr Tyr Gly
     50                  55                  60

tgt gag gga cca ggt ctt gaa ggc tgt gca aaa cct ggg ccc aag atc    240
Cys Glu Gly Pro Gly Leu Glu Gly Cys Ala Lys Pro Gly Pro Lys Ile
 65                  70                  75                  80

cca tcc att gct acc ggg att gtc ggc ggc ctc ctc ttg gtg gtg gtg    288
Pro Ser Ile Ala Thr Gly Ile Val Gly Gly Leu Leu Leu Val Val Val
                 85                  90                  95

gtg gcc ctt gga gtg ggc ctc ttt ttg cgc cgg cgc cac att gtc cgg    336
Val Ala Leu Gly Val Gly Leu Phe Leu Arg Arg Arg His Ile Val Arg
            100                 105                 110

aag cgc acg ctt cgc aga ctg ctg caa gaa aga gag ctt gtc gag cct     384
Lys Arg Thr Leu Arg Arg Leu Leu Gln Glu Arg Glu Leu Val Glu Pro
        115                 120                 125

ctt aca ccc agc gga gaa gct ccc aac cag gct ctc ttg agg atc tta     432
Leu Thr Pro Ser Gly Glu Ala Pro Asn Gln Ala Leu Leu Arg Ile Leu
    130                 135                 140

aag gag acg gag ttc aaa aag atc aag gtg ctg ggc tct gga gca ttc     480
Lys Glu Thr Glu Phe Lys Lys Ile Lys Val Leu Gly Ser Gly Ala Phe
145                 150                 155                 160

ggc acg gtg tat aag gga ctc tgg atc cca gaa ggc gag aag gtt aaa     528
Gly Thr Val Tyr Lys Gly Leu Trp Ile Pro Glu Gly Glu Lys Val Lys
                165                 170                 175

att ccc gtg gcc atc aag gaa ttg aga gaa gcc acg tct ccc aaa gcc     576
Ile Pro Val Ala Ile Lys Glu Leu Arg Glu Ala Thr Ser Pro Lys Ala
            180                 185                 190

aac aag gaa att ctt gat gaa gcc tac gtg atg gcc agt gtg gac aat     624
Asn Lys Glu Ile Leu Asp Glu Ala Tyr Val Met Ala Ser Val Asp Asn
        195                 200                 205

ccc cac gtg tgc cgc ctc ctg ggc atc tgc ctg acg tcc acg gtg cag     672
Pro His Val Cys Arg Leu Leu Gly Ile Cys Leu Thr Ser Thr Val Gln
    210                 215                 220

ctc atc acg cag ctc atg ccc ttt ggc tgc ctc ctg gac tat gtc cgc     720
Leu Ile Thr Gln Leu Met Pro Phe Gly Cys Leu Leu Asp Tyr Val Arg
225                 230                 235                 240

gag cac aag gac aac atc ggc tcc cag cac ctg ctc aac tgg tgt gtg     768
Glu His Lys Asp Asn Ile Gly Ser Gln His Leu Leu Asn Trp Cys Val
                245                 250                 255

cag att gca aag ggc atg aac tac ctg gaa gac cgg cgc ttg gtg cac     816
Gln Ile Ala Lys Gly Met Asn Tyr Leu Glu Asp Arg Arg Leu Val His
            260                 265                 270

cgc gac ctg gcg gcc agg aac gtc ctg gtg aag acc ccg cag cac gtc     864
Arg Asp Leu Ala Ala Arg Asn Val Leu Val Lys Thr Pro Gln His Val
        275                 280                 285

aag atc aca gat ttt ggg ctg gcc aaa ctg ctg ggt gcc gag gag aaa     912
Lys Ile Thr Asp Phe Gly Leu Ala Lys Leu Leu Gly Ala Glu Glu Lys
    290                 295                 300

gag tac cac gcg gaa gga ggc aaa gtg cct atc aag tgg atg gct tta     960
Glu Tyr His Ala Glu Gly Gly Lys Val Pro Ile Lys Trp Met Ala Leu
305                 310                 315                 320

gaa tcg att tta cac cga att tat acc cac caa agc gat gtg tgg agc    1008
Glu Ser Ile Leu His Arg Ile Tyr Thr His Gln Ser Asp Val Trp Ser
                325                 330                 335

tac ggc gtc acc gtg tgg gag ctg atg acc ttc ggg tcc aag cct tac     1056
Tyr Gly Val Thr Val Trp Glu Leu Met Thr Phe Gly Ser Lys Pro Tyr
            340                 345                 350

gac ggt atc cct gca agt gag atc tcc acc atc ctg gag aag gga gag     1104
Asp Gly Ile Pro Ala Ser Glu Ile Ser Thr Ile Leu Glu Lys Gly Glu
        355                 360                 365

cgc ctc ccg cag ccg ccc ata tgc acc atc gat gtc tac atg atc atg     1152
Arg Leu Pro Gln Pro Pro Ile Cys Thr Ile Asp Val Tyr Met Ile Met
    370                 375                 380

gtc aag tgc tgg atg ata gat gca gac agt cgc cca aaa ttc cgt gag     1200
Val Lys Cys Trp Met Ile Asp Ala Asp Ser Arg Pro Lys Phe Arg Glu
385                 390                 395                 400

ttg atc atc gaa ttc tcc aaa atg gcc cga gac ccg cag cgc tac ctt     1248
Leu Ile Ile Glu Phe Ser Lys Met Ala Arg Asp Pro Gln Arg Tyr Leu
                405                 410                 415

gtc atc cag gga gat gag agg atg cat ttg cca agc cct aca gac tcc     1296
Val Ile Gln Gly Asp Glu Arg Met His Leu Pro Ser Pro Thr Asp Ser
            420                 425                 430

aat ttt tac cgc gcc ctg atg gac gag gag gac atg gag gat gtt gtg     1344
Asn Phe Tyr Arg Ala Leu Met Asp Glu Glu Asp Met Glu Asp Val Val
        435                 440                 445

gat gct gac gag tac ctc atc ctc cag cag ggc ttc ttc cac agc ccc     1392
Asp Ala Asp Glu Tyr Leu Ile Leu Gln Gln Gly Phe Phe His Ser Pro
    450                 455                 460

tcc act tcc cgg acc ccc ctc cta agt tct ctg agc gcc acc agc aac     1440
Ser Thr Ser Arg Thr Pro Leu Leu Ser Ser Leu Ser Ala Thr Ser Asn
465                 470                 475                 480

agt tcc aac gtg gct tgc atc gac cga aat ggg acc tgt ccc ctc aaa     1488
Ser Ser Asn Val Ala Cys Ile Asp Arg Asn Gly Thr Cys Pro Leu Lys
                485                 490                 495

gaa gac agc ttc ttg cag cgg tac agc tca gac ccc act ggc acc ttg     1536
Glu Asp Ser Phe Leu Gln Arg Tyr Ser Ser Asp Pro Thr Gly Thr Leu
            500                 505                 510

acg gag gac aac ata gat gac act ttc ctc cca gca ccc gaa tac ata     1584
Thr Glu Asp Asn Ile Asp Asp Thr Phe Leu Pro Ala Pro Glu Tyr Ile
        515                 520                 525

aac cag tct gtt ccc aaa agg cct gcg ggt tct gtc cag aat ccc gtc     1632
Asn Gln Ser Val Pro Lys Arg Pro Ala Gly Ser Val Gln Asn Pro Val
    530                 535                 540

tat cac aat cag cct cta aat cca gct cct gcc aga gac cct cac tac     1680
Tyr His Asn Gln Pro Leu Asn Pro Ala Pro Ala Arg Asp Pro His Tyr
545                 550                 555                 560

caa aat ccc cac agc aac gca gtg gac aac cct gag tat ctc aac acc     1728
Gln Asn Pro His Ser Asn Ala Val Asp Asn Pro Glu Tyr Leu Asn Thr
                565                 570                 575

cac ccc acc tgc gtc aac agt gtg ctc gac agg ccc agc ctc tgg acc     1776
His Pro Thr Cys Val Asn Ser Val Leu Asp Arg Pro Ser Leu Trp Thr
            580                 585                 590

cag gag gcc aac cac caa atc agc ctg gac aac cct gac tac cag cag     1824
Gln Glu Ala Asn His Gln Ile Ser Leu Asp Asn Pro Asp Tyr Gln Gln
        595                 600                 605

gac ttc ttt ccc aag gaa gcc aag tcc aat ggc att ttt aag ggc cct     1872
Asp Phe Phe Pro Lys Glu Ala Lys Ser Asn Gly Ile Phe Lys Gly Pro
    610                 615                 620

gca gct gaa aat gca gac tac ctg agg gta gcg cca cca agc agt gag     1920
Ala Ala Glu Asn Ala Asp Tyr Leu Arg Val Ala Pro Pro Ser Ser Glu
625                 630                 635                 640

ttt att gga gca tga                                                 1935
Phe Ile Gly Ala
                645

<210> 2
<211> 644
<212> PRT
<213> Canis familiaris

<400> 2
Pro Gln Ala Met Asn Ile Thr Cys Thr Gly Arg Gly Pro Asp Ser Cys
  1               5                  10                  15

Ile Lys Cys Ala His Tyr Ile Asp Gly Pro His Cys Val Lys Thr Cys
             20                  25                  30

Pro Ala Gly Ile Met Gly Glu Asn Asn Thr Leu Val Trp Lys Phe Ser
         35                  40                  45

Asp Gly Ser Arg Met Cys His Leu Cys His Pro Asn Cys Thr Tyr Gly
     50                  55                  60

Cys Glu Gly Pro Gly Leu Glu Gly Cys Ala Lys Pro Gly Pro Lys Ile
 65                  70                  75                  80

Pro Ser Ile Ala Thr Gly Ile Val Gly Gly Leu Leu Leu Val Val Val
                 85                  90                  95

Val Ala Leu Gly Val Gly Leu Phe Leu Arg Arg Arg His Ile Val Arg
            100                 105                 110

Lys Arg Thr Leu Arg Arg Leu Leu Gln Glu Arg Glu Leu Val Glu Pro
        115                 120                 125

Leu Thr Pro Ser Gly Glu Ala Pro Asn Gln Ala Leu Leu Arg Ile Leu
    130                 135                 140

Lys Glu Thr Glu Phe Lys Lys Ile Lys Val Leu Gly Ser Gly Ala Phe
145                 150                 155                 160

Gly Thr Val Tyr Lys Gly Leu Trp Ile Pro Glu Gly Glu Lys Val Lys
                165                 170                 175

Ile Pro Val Ala Ile Lys Glu Leu Arg Glu Ala Thr Ser Pro Lys Ala
            180                 185                 190

Asn Lys Glu Ile Leu Asp Glu Ala Tyr Val Met Ala Ser Val Asp Asn
        195                 200                 205

Pro His Val Cys Arg Leu Leu Gly Ile Cys Leu Thr Ser Thr Val Gln
    210                 215                 220

Leu Ile Thr Gln Leu Met Pro Phe Gly Cys Leu Leu Asp Tyr Val Arg
225                 230                 235                 240

Glu His Lys Asp Asn Ile Gly Ser Gln His Leu Leu Asn Trp Cys Val
                245                 250                 255

Gln Ile Ala Lys Gly Met Asn Tyr Leu Glu Asp Arg Arg Leu Val His
            260                 265                 270

Arg Asp Leu Ala Ala Arg Asn Val Leu Val Lys Thr Pro Gln His Val
        275                 280                 285

Lys Ile Thr Asp Phe Gly Leu Ala Lys Leu Leu Gly Ala Glu Glu Lys
    290                 295                 300

Glu Tyr His Ala Glu Gly Gly Lys Val Pro Ile Lys Trp Met Ala Leu
305                 310                 315                 320

Glu Ser Ile Leu His Arg Ile Tyr Thr His Gln Ser Asp Val Trp Ser
                325                 330                 335

Tyr Gly Val Thr Val Trp Glu Leu Met Thr Phe Gly Ser Lys Pro Tyr
            340                 345                 350

Asp Gly Ile Pro Ala Ser Glu Ile Ser Thr Ile Leu Glu Lys Gly Glu
        355                 360                 365

Arg Leu Pro Gln Pro Pro Ile Cys Thr Ile Asp Val Tyr Met Ile Met
    370                 375                 380

Val Lys Cys Trp Met Ile Asp Ala Asp Ser Arg Pro Lys Phe Arg Glu
385                 390                 395                 400

Leu Ile Ile Glu Phe Ser Lys Met Ala Arg Asp Pro Gln Arg Tyr Leu
                405                 410                 415

Val Ile Gln Gly Asp Glu Arg Met His Leu Pro Ser Pro Thr Asp Ser
            420                 425                 430

Asn Phe Tyr Arg Ala Leu Met Asp Glu Glu Asp Met Glu Asp Val Val
        435                 440                 445

Asp Ala Asp Glu Tyr Leu Ile Leu Gln Gln Gly Phe Phe His Ser Pro
    450                 455                 460

Ser Thr Ser Arg Thr Pro Leu Leu Ser Ser Leu Ser Ala Thr Ser Asn
465                 470                 475                 480

Ser Ser Asn Val Ala Cys Ile Asp Arg Asn Gly Thr Cys Pro Leu Lys
                485                 490                 495

Glu Asp Ser Phe Leu Gln Arg Tyr Ser Ser Asp Pro Thr Gly Thr Leu
            500                 505                 510

Thr Glu Asp Asn Ile Asp Asp Thr Phe Leu Pro Ala Pro Glu Tyr Ile
        515                 520                 525

Asn Gln Ser Val Pro Lys Arg Pro Ala Gly Ser Val Gln Asn Pro Val
    530                 535                 540

Tyr His Asn Gln Pro Leu Asn Pro Ala Pro Ala Arg Asp Pro His Tyr
545                 550                 555                 560

Gln Asn Pro His Ser Asn Ala Val Asp Asn Pro Glu Tyr Leu Asn Thr
                565                 570                 575

His Pro Thr Cys Val Asn Ser Val Leu Asp Arg Pro Ser Leu Trp Thr
            580                 585                 590

Gln Glu Ala Asn His Gln Ile Ser Leu Asp Asn Pro Asp Tyr Gln Gln
        595                 600                 605

Asp Phe Phe Pro Lys Glu Ala Lys Ser Asn Gly Ile Phe Lys Gly Pro
    610                 615                 620

Ala Ala Glu Asn Ala Asp Tyr Leu Arg Val Ala Pro Pro Ser Ser Glu
625                 630                 635                 640

Phe Ile Gly Ala

<210> 3
<211> 19
<212> DNA
<213> Artificial Sequence

<220>
<223> Description of Artificial Sequence: Primer

<400> 3
cctcaggcca tgaacatca                                                19

<210> 4
<211> 21
<212> DNA
<213> Artificial Sequence

<220>
<223> Description of Artificial Sequence: Primer

<400> 4
ttgatgaagc ctacgtgatg g                                                21

<210> 5
<211> 20
<212> DNA
<213> Artificial Sequence

<220>
<223> Description of Artificial Sequence: Primer

<400> 5
cgttcggcac ggtgtataag                                                  20

<210> 6
<211> 23
<212> DNA
<213> Artificial Sequence

<220>
<223> Description of Artificial Sequence: Primer

<400> 6
aaatgcattc tttcatcccc ctg                                              23

<210> 7
<211> 24
<212> DNA
<213> Artificial Sequence

<220>
<223> Description of Artificial Sequence: Primer

<400> 7
tacatgatca tggtcaagtg ctgg                                             24

<210> 8
<211> 23
<212> DNA
<213> Artificial Sequence

<220>
<223> Description of Artificial Sequence: Primer

<400> 8
ctccgtggtc atgctccaat aaa                                              23

<210> 9
<211> 20
<212> DNA
<213> Artificial Sequence

<220>
<223> Description of Artificial Sequence: Primer

<400> 9
ccccagggag tatgtgaatg                                                    20


<210> 10
<211> 17
<212> DNA
<213> Artificial Sequence

<220>
<223> Description of Artificial Sequence: Primer

<400> 10
gctgggccca agtcctc                                                       17


<210> 11
<211> 22
<212> DNA
<213> Artificial Sequence

<220>
<223> Description of Artificial Sequence: Primer

<400> 11
tcaagtggat ggcgctggag tc                                                 22


<210> 12
<211> 20
<212> DNA
<213> Artificial Sequence

<220>
<223> Description of Artificial Sequence: Primer

<400> 12
aggaggaggg tggggctgag                                                    20


<210> 13
<211> 24
<212> DNA
<213> Artificial Sequence

<220>
<223> Description of Artificial Sequence: Primer

<400> 13
cacatgaccc cagccctcta cagc                                              24

<210> 14
<211> 24
<212> DNA
<213> Artificial Sequence

<220>
<223> Description of Artificial Sequence: Primer

<400> 14
cccccacact tgcctcccca taca                                              24

<210> 15
<211> 20
<212> DNA
<213> Artificial Sequence

<220>
<223> Description of Artificial Sequence: Primer

<400> 15
gccaaaaggg tcatcatctc                                                   20

<210> 16
<211> 18
<212> DNA
<213> Artificial Sequence

<220>
<223> Description of Artificial Sequence: Primer

<400> 16
ggccatccac agtcttct                                                     18

<210> 17
<211> 20
<212> DNA
<213> Artificial Sequence

<220>
<223> Description of Artificial Sequence: Primer

<400> 17
cgttgctatc caggctgtgc                                                   20

<210> 18
<211> 20
<212> DNA
<213> Artificial Sequence

<220>
<223> Description of Artificial Sequence: Primer

<400> 18
gtagtttcgt ggatgccaca                                                    20

<210> 19
<211> 36
<212> DNA
<213> Artificial Sequence

<220>
<223> Description of Artificial Sequence: Primer

<400> 19
attcgcgact gatgatcgat tttttttttt tttttt                                  36

<210> 20
<211> 27
<212> DNA
<213> Artificial Sequence

<220>
<223> Description of Artificial Sequence: Primer

<400> 20
tacggctgcg agaagacgac agaaggg                                            27

<210> 21
<211> 22
<212> DNA
<213> Artificial Sequence

<220>
<223> Description of Artificial Sequence: Primer

<400> 21
aactacctgg aagaccggcg ct                                                 22

<210> 22
<211> 24
<212> DNA
<213> Artificial Sequence

<220>
<223> Description of Artificial Sequence: Primer

<400> 22
tacggctgcg agaagacgac agaa                                              24

## Description

The present invention relates generally to the treatment of cancer in animals, particularly to the treatment of mammary tumors in dogs.

Cancer of the mammary gland in the dog has shown to be approximately as common as it is in humans. Mammary tumors are the most common tumors of the female dog, and also the most common malignant ones (Walter et al., 1997; Walter and Schwegler, 1992; Bonnet et al., 1997).

Presently, in the treatment of canine mammary tumors, the surgical removal of the tumor is the method of choice. If surgery is done early in the course of this disease, the cancer can, in most cases, be totally eliminated in over 50% of the cases showing a malignant form of cancer.

While the effectiveness of radiation therapy for the treatment of dogs has not been thoroughly researched, several human anti-cancer drugs such as doxorubicin and actinomycin have been used in dogs. Also, some anti-hormonal drug regimens are being tested in dogs.

It was an object of the invention to provide new pharmaceutical compositions for the treatment of cancers in non-human mammals, in particular for the treatment of mammary tumors in dogs.

Modjtahedi and Dean (1994) have shown that excess EGFR (EGF receptor, Epidermal growth factor receptor) activity directly contributes to tumor cell transformation. Due to this finding, overexpressed growth factor receptors with tyrosine kinase activity, e.g. EGFR, have been recognized as promising targets for tumor therapy in humans (Woodburn, 1999; Levitzky, 1999; Klohs et al., 1997).

Consequently, chemical compounds that inhibit the tyrosine kinase activity of the growth factor receptors, antibodies directed to the extracellular domain of a growth factor receptor or the growth factors (or active fragments thereof) themselves have been suggested for the therapy of human cancers (Woodburn, 1999).

Davies and Chamberlin (1996) and Levitzky (1994), report that small molecules, which are selective for the EGFR and have the ability to inhibit the intracellular kinase domain of the EGFR, are capable of reverting the aberrant enzymatic activity of the EGFR in tumor cells. An example of a substance, which belongs to the class of pyrimido-pyrimidines, is BIBX1382: 4-((3-chloro-4-fluoro-phenyl)amino)-6-(1-methyl-4-piperidinyl-amino)-pyrimido(5,4d)pyrimidine).

It was shown that the EGFR is present in canine mammary tumors and correlates with other receptors, e.g. the estrogen receptor (Nerurkar et al., 1987). Although it was shown that mammary tumors express receptors that bind EGF the sequence of the canine EGFR was not known.

To solve the problem underlying the present invention, based on the knowledge about aberrant tyrosine kinase receptor activity in humans and the inhibition thereof in human cancer therapy, the utility of tyrosine kinase receptor inhibitors for the therapy of certain non-human mammalian cancers, in particular for the therapy of mammary tumors of the dog, was assessed.

To this end, in a first step, the canine EGF receptor was cloned and sequenced.

It was found that the human and the canine EGFR exhibit a high degree of identity. Therefore, in a next step, it was tested whether inhibitors of the human EGF receptor are capable of inhibiting the canine EGF receptor and may thus be used in the therapy of cancers with aberrant expression of this receptor.

In the experiments of the present invention, it was found that a tyrosine kinase receptor inhibitor that has been suggested for the treatment of human malignancies, is effective for inhibiting canine mammary tumors.

Therefore, the present invention relates to a pharmaceutical composition for the treatment of mammary tumors of a non-human mammal, which is associated with an aberrant activity of one or more tyrosine kinase receptors, wherein said pharmaceutical composition contains, as the active ingredient, one or more substances that inhibit the aberrant activity of said tyrosine kinase receptor(s).

In a preferred embodiment, the pharmaceutical composition of the invention is used for the therapy of mammary tumors in dogs, however, it is also useful for the treatment of mammary tumors of other mammals. Since the kinase domains of the EGFRs in various mammals have shown to exhibit 100% identity on the amino acid level, the pharmaceutical composition of the invention can be applied to other animals, e.g. cats, provided there is a therapeutic need and provided the tyrosine kinase receptor that is to be inhibited, in particular the EGFR, has a high degree of identity with the corresponding human receptor. Preferably, the composition can be used for the treatment of animals whose tyrosine kinase receptor, in particular the EGFR, has an identity of 100% in the ATP binding pocket, more preferably, an identity of 100 % in the entire kinase domain. The identity of the tyrosine kinase receptor domains between the animal of interest and man, or another mammal whose tyrosine kinase receptor has been cloned and sequenced, can be readily determined by cloning the receptor, as described for the canine EGFR in Example 1.

In the present invention "aberrant activity" of the tyrosine kinase receptor, in particular the EGFR, in tumor cells of epithelial origin is defined as a) overexpression of the EGFR from the gene, b) expression of the EGFR from the amplified gene, c) expression of the EGFR from a mutated version of the gene, or d) increased sensitivity of tumor cells to the inhibition of EGFR signaling (it has been suggested that tumor cells may have an increased sensitivity to the inhibition of their major receptor tyrosine kinase's pathway).

In a preferred embodiment, the active substance is an inhibitor of a class I tyrosine kinase receptor.

In a particularly preferred embodiment, the active substance is an EGF receptor inhibitor.

Since the EGF receptor was shown to be present in the majority of canine mammary tumor samples (Nerurkar et al., 1987), it can serve as a biochemical marker for canine mammary tumors. Based on the sequence of the canine EGFR, immunohistochemistry or Northern Blot analysis can be used as diagnostic tools for detection of EGF receptor overexpression in canine tumor tissue samples. This allows for prediction and monitoring of the therapeutic benefit of an EGF receptor inhibitor therapy.

HER2 expression, besides expression of other receptor tyrosine kinases, has been shown to be causally involved in breast cancer (Baselga and Mendelsohn, 1997). Aberrant expression of HER2, like EGFR, in advanced breast cancer correlates with poor prognosis, and the anti-HER2 antibody trastuzumab (Herceptin) is being considered as one of the most promising therapeutic agents for the treatment of breast cancer.

Earp et al., 1995, have shown that members of the tyrosine kinase class I receptors can form heterodimers (e.g. between EGFR and HER2). Dimerization is the initial step in tyrosine kinase activation. Furthermore, these authors have shown that sequence homology on the amino acid level between the kinase domains of HER2, HER3 and HER4 and the kinase domain of EGFR is 82%, 59% and 79%, respectively.

For these reasons, an EGFR inhibitor has the ability not only to inhibit signaling through an EGFR homodimer, but also through EGFR/HER2 or other heterodimers.

Furthermore, specific inhibition of HER2, alone or in combination with inhibition of EGFR, provides a therapy that covers a broader spectrum of tumors.

Therefore, in another preferred embodiment, the active substance is an inhibitor of a tyrosine kinase receptor selected from HER2, HER3 and HER4, preferably HER2, e.g. the anti-HER2-antibody trastuzumab (Herceptin) or a specific HER2 inhibitor as described in US 5,721,237.

In analogy with the canine EGFR, the canine HER2 is cloned and sequenced to confirm the homology between the human and the canine proteins.

In the preferred embodiment, chemical compounds useful as the active ingredient in the pharmaceutical composition of the invention are inhibitors of the corresponding human tyrosine kinase receptors, e.g. the human EGFR.

Examples of EGFR low molecular weight inhibitors have been described by Strawn and Shawver, 1998, and McMahon et al., 1998. Examples of tyrosine kinase inhibitors are also described in, *inter alia,* EP 682 027, WO 95/19970, WO 96/07657, WO 96/33980, e.g. N-(3-chloro-4-fluorophenyl)-7-methoxy-6-[3-(4-morpholinyl)propoxy]-4-chinazolinamine (ZD-1839); WO 96/30347, e.g. N-(3-ethinylphenyl)-6,7-bis(2-methoxyethoxy)-4-chinazolinamine (CP 358774); WO 97/38983, e.g. N-(4-(3-(chloro-4-fluoro-phenylamino-7-(3-morpholine-4-yl-propoxy)-chinazoline-6-yl)-acrylamiddihydrochloride (CI 1033, PD 183805); WO 97/02266 (phenylaminopyrrolopyrimidine; PKI-166); examples of protein kinase inhibitors are also given in the article by Mc Mahon et al., 1998.

Among the tyrosine kinase inhibitors known in the art, ATP site-directed inhibitors are particularly useful in the present invention.

Preferably, the inhibitor is a pyrimido-pyrimidine.

In a particularly preferred embodiment, the pyrimido pyrimidine is 4-((3-chloro-4-fluoro-phenyl)amino)-6-(1-methyl-4-piperidinyl-amino)-pyrimido(5,4d)pyrimidine (which is the compound BIBX1382 used in the Examples).

The tyrosine kinase inhibitor may be administered either on its own or in conjunction with one or more other active substances. For example, a compound specifically inhibiting a certain member of a class I tyrosine kinase receptor may be combined with an inhibitor of another representative of that receptor class. Alternatively, or in addition, the tyrosine kinase inhibitor(s) may be used in conjunction with other pharmacologically active compounds, in particular with other anti-tumor therapeutic agents, for example in combination with topoisomerase inhibitors (e.g. etoposide), mitosis inhibitors (e.g. vinblastin), compounds which interact with nucleic acids (e.g. cis-platin, cyclophosphamide, adriamycin), hormone antagonists (e.g. tamoxifen), inhibitors of metabolic processes (e.g. 5-FU, gemcitabine etc.), cytokines (e.g. interferons), antibodies, etc.

The pharmaceutical composition of the invention may be administered by the intravenous, subcutaneous, intramuscular, intrarectal, intraperitoneal or intranasal route, by inhalation or transdermally or orally.

The tyrosine kinase inhibitors are generally used in doses of 0.01-100 mg/kg of body weight, preferably 0.1-15 mg/kg. For administration, the compounds are formulated with one or more conventional inert carriers and/or diluents, e.g. with corn starch, lactose, glucose, microcrystalline cellulose, magnesium stearate, polyvinylpyrrolidone, citric acid, tartaric acid, water, water/ethanol, water/glycerol, water/sorbitol, water/polyethyleneglycol, propyleneglycol, stearylalcohol, carboxymethylcellulose or fatty substances such as hard fat or suitable mixtures thereof in conventional galenic preparations such as plain or coated tablets, capsules, powders, suspensions, solutions, sprays or suppositories.

### Brief description of the figures:

- Fig. 1:: Strategy for cloning the canine EGFR
- Fig. 2:: Expression of the EGFR in various canine cell lines (Northern Blot)
- Fig. 3:: Inhibition of the proliferation of cells in culture by the EGFR inhibitor BIBX1382 (³H-Thymidin proliferation assay)
- Figs. 4 and 5:: Effect of the EGFR inhibitor BIBX1382 on tumor growth in nude mice
- Fig. 6:: Detection of the canine EGF receptor by immunohistochemical staining of paraffin sections of canine mamma-adenocarcinomas with a human EGFR-antibody
- Fig. 7:: Detection of EGFR on the cellular level by cytospin analysis

### Example 1

Cloning of the canine EGF receptor

For the design of the strategy to clone the canine EGF receptor the major part of the intracellular domain, the whole transmembrane and a small part of the extracellular domain of the human EGFR-sequence were used.

### a) Preparation of RNA

The cell line CCL-34 was purchased from ATCC. It was derived from a kidney of an apparently normal adult female dog. It was cultured in T25, T75 or T175 tissue culture bottles (Greiner) at 37°C and 5% CO2. The medium was Dulbecco's Modified Eagle's Medium (DMEM) supplemented with 10% fetal calf serum (FCS, PAA), non-essentiell amino acids (GIBCO BRL) and a standard complement of antibiotics (penicillin/streptomycin, GIBCO BRL). Cultures were split with trypsin EDTA (GIBCO BRL) when they were subconfluent. The split ration was 1:2 to 1:10.

The day before RNA preparation was performed, the cells were split with a ratio of 1:2. The next day the cells were lysed directly in the culture dish by adding TRIzol Reagent (GIBCO BRL) and the RNA was isolated using the protocol provided with the reagent. After isolation the amount of RNA was measured with a photometer and the RNA was stored at -80°C.

### b) First strand cDNA synthesis

cDNA synthesis of 1µg CCL-34 RNA was done by using Superscript II Rnase H Reverse Transcriptase (GIBCO BRL) according to the standard protocol provided with the components. As a control, GAPDH and β-Actin PCR were performed.
- GAPDH-primer:: SEQ ID NO:15(sense)
SEQ ID NO:16 (antisense)
- β-Actin-primer:: SEQ ID NO:17 (sense)
SEQ ID NO:18(antisense)

The PCR was run for a total of 35 cycles at the following temperatures and times: 95°C (30 seconds) and 58°C (1 minute 30 seconds). The GAPDH PCR resulted in a 229bp band and the β-Actin in a 430bp fragment on the agarose gel.

### c) Primers and PCR reactions

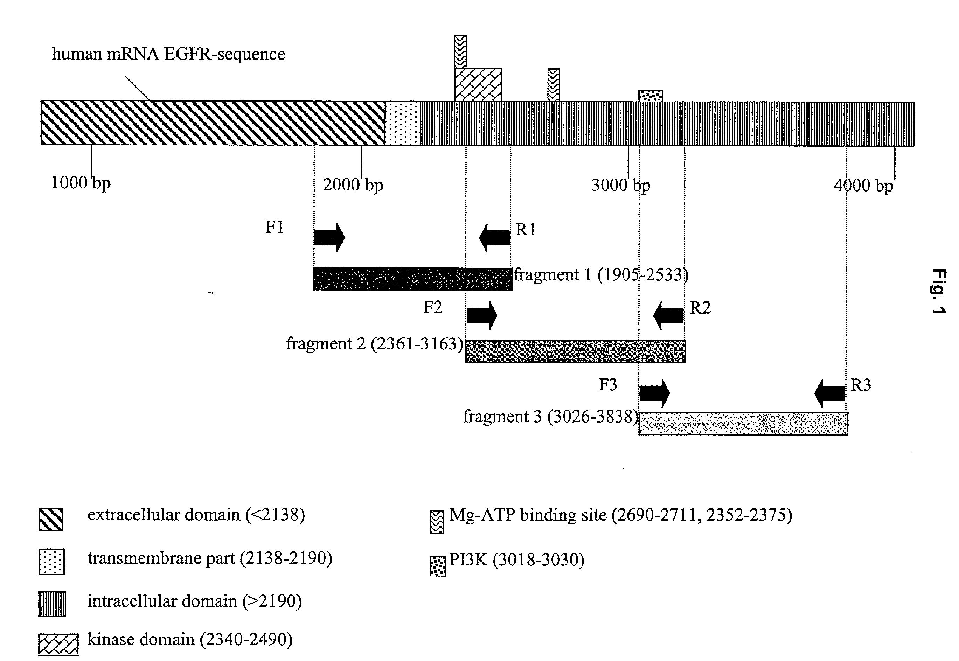

Next, three overlapping PCR primer pairs were designed to encompass the major part of the intracellular domain, the whole transmembrane and a small part of the extracellular domain of the human EGFR-sequence (the cloning strategy and the fragments are depicted in Fig. 1) :
- Fragment 1:: SEQ ID NO:3(F1test, sense)
SEQ ID NO:4(R1test, antisense)
- Fragment 2:: SEQ ID NO:5 (F2,sense)
SEQ ID NO:6 (R2, antisense)
- Fragment 3:: SEQ ID NO:7(F3, sense)
SEQ ID NO:8(R3, antisense)

The PCR for fragment 1 was run for a total of 35 cycles at the following temperatures and times: 95°C (30 seconds), 56°C (30 seconds) and 72°C (1 minute). This PCR resulted in the fragment 1 with the length of 594bp. To produce fragment 2 the PCR was run for a total of 40 cycles at the following temperatures and times: 95°C (30 seconds), 58°C (30 seconds) and 72°C (1 minute). Fragment 2 showed a length of ∼800bp on the agarose gel. Fragment 3 was amplified in a PCR reaction with a total of 35 cycles at following temperatures and times: 95°C (30 seconds), 59°C (30 seconds) and 72°C (1 minute). The resulting fragment had a length of ∼800bp pairs.

### d) Subcloning of the PCR fragments and transformation

After the PCR reactions the resulting fragments were cloned into the TA-vector-system. The ligation and the transformation was done by using the protocol provided with the TA-vector system components. Blue-white selection was performed, the white colonies contain plasmids with an insert.

### e) Isolation of plasmids

The white colonies were picked from the plates and inoculated in LB-Ampicillin medium over night at 37°C and 225 rpm. The bacteria suspension was taken and the plasmids were isolated using a standard protocol for alkaline lysis. After ethanol precipitation the plasmid-DNA was dissolved in water. Control restriction digests were performed using the restriction enzymes Spe I and Eco RV (Promega). The vectors and inserts were analysed on an agarose gel to check the length of the fragments 1, 2 and 3.

### f) Sequencing procedures

With those plasmids that contained inserts with the right length PCR for sequencing was performed. The three primer pairs F1test/R1test, F2/R2 and F3/R3 (see c)) were again used for these PCR reactions which were run for a total of 25 cycles at the following temperatures and times: 96°C (30 seconds), 50°C (15 seconds) and 60°C (4 minutes). The PCR products were precipitated with ethanol, washed, air-dried and sequenced.

### g) Comparison of the nucleotide and amino acid sequences

The comparison between the achived three fragments and the human EGFR-mRNA-sequence was done by using the computer program "Megalign".

### h) Cloning of the 5'-end of the canine EGF-receptor

For the cloning of the 5'-end of the canine EGF-receptor the SMART (Switch mechanism at 5'-end of RNA templates) cDNA synthesis technology was used. SMART cDNA synthesis started with total RNA of the cell line CCL34.

Following primers were used for this method:
SEQ ID NO:19 (oligo(dT))
SEQ ID NO:20 (cDNA-ON)
SEQ ID NO:21 (R1b)
SEQ ID NO:22 (PCR-ON)
cDNA synthesis was performed using oligo(dT) and cDNA-ON primers according to a standard protocol. After this synthesis, a PCR reaction was carried out with the primers R1b and PCR-ON. The PCR was run for 30 cycles at the following temperatures and times: 94°C (30 seconds) and 68°C (3 minutes). The PCR product was analysed on an agarose gel, which was then used for Southern Blotting. The blotting onto nylon membrane was done by using a standard protocol. Fragment 1 from the canine EGF-receptor was labeled and the membrane was hybridized over night. Again, a PCR reaction was performed with these primers and analysed on gel. According to the bands, that appeared after scanning the blot, the bands were cut out of the gel. The DNA was isolated, ligated into the TA-vector and transformed into one shot competent cells using the protocol provided with the TA-vector-system. After plasmid isolation with alkaline lyses the inserts in the plasmids were sequenced.

Tables 1 and 2 show the sequence comparison of the subcloned fragments and the human EGFR-sequence, tables 3 and 4 show the comparison of the complete EGFR-sequences, or the kinase domains respectively, between different species.

**Table 1**

| Homology between the canine and human EGFR sequence | | |
|---|---|---|
| fragment | similarity index in DNA-sequence | similarity index in amino acid sequence |
| 1 | 85,9% | 90,4% |
| 2 | 91,5% | 99,3% |
| 3 | 86,7% | 87,9% |

**Table 2**

| Comparison of the DNA and amino acid level of various fragments from domains of the human and canine EGFR | | | |
|---|---|---|---|
| fragment | domain | similarity index | |
| | | DNA level | amino acid level |
| 1 | kinase domain | 91,5% | 100,0% |
| 2 | kinase domain | 92,3% | 100,0% |
| 2 | Mg-ATP binding site (1) | 100,0% | 100,0% |
| 2 | Mg-ATP binding site (2) | 100,0% | 100,0% |
| 2 | PI3K | 100,0% | 100,0% |

**Table 3**

| Comparison of the EGFR sequences between different species | | |
|---|---|---|
| | DNA level | amino acid level |
| rat/mice | 87,6% | 95,6% |
| canine/rat | 79,1% | 90,4% |
| canine/mice | 81,9% | 90,1% |

**Table 4**

| Comparison of the EGFR kinase domains between different species, based on the articles by Ullrich et al., 1984; Peth et al., 1990; Luetteke et al., 1994. | | |
|---|---|---|
| | DNA-sequence | amino acid sequence |
| human/mice | 86,8% | 100% |
| human/rat | 88,1% | 100% |
| human/canine | 90,7% | 100% |

As can be seen, homology on the DNA level between human and dog is the highest; there are no differences on the amino acid level.

### Example 2

Analysis of EGFR expression and cell proliferation; inhibitory effect of an inhibitor of human EGFR (BIBX1382) on canine tumor cell proliferation

To analyse if the EGF receptor is expressed in different tumor cell lines (see below) and whether there is a correlation between its expression and an inhibition of cell proliferation by BIBX1382, an expression analysis on Northern Blot and by PCR and a ³H-Thymidin proliferation assay were performed:

### a) EGFR expression analysis by Northern Blot

The cell lines SLU-1 (Hellmén, 1992 and 1993; van Leeuwen et al., 1996), SLU-2 (Hellmén, 2000), SLU-3 (Hellmén, 1992 and 1993), SLU-4 (Hellmén, 1992 and 1993; van Leeuwen et al., 1996) and T5 were cultured in T25 or T75 tissue culture bottles at 37°C and 5% CO₂. The medium was RPMI (Gibco cat # 21980-32) supplemented with 10% FCS (PAA) and standard antibiotics. Cultures were split at ratios of between 1:3 and 1:10.

The tumor cells T5 were isolated from a fresh canine mixed mamma-tumor. The tumor was cut down into small pieces, picked up in medium and was run through a cell strainer. The flow through was centrifuged for ten minutes at 200-250g, resuspended and again centrifuged. Cells were suspended in medium and were cultured in a T25 tissue culture bottle. Afterwards the cells were cultured in T25 or T75 tissue culture bottles (Greiner) at 37°C and 5% CO₂. The medium was RPMI supplemented with 10% fetal calf serum (FCS, PAA), non-essential amino acids (GIBCO BRL) and a standard complement of antibiotics (penicillin/streptomycin, GIBCO BRL). Cultures were split with Trypsin-EDTA (GIBCO BRL) when they were subconfluent. The split ration was 1:2.

The tumor cell lines p114 (van Leeuwen et al., 1996) and SH1b (van der Burg et al., 1989; van Leeuwen et al., 1996) were also cultured in T25, T75 or T175 tissue culture bottles (Greiner) at 37°C and 5% CO2. The medium was again Dulbecco's Modified Eagle's Medium (DMEM) supplemented with 10% fetal calf serum (FCS, PAA), non-essentiell amino acids (GIBCO BRL) and a standard complement of antibiotics (penicillin/streptomycin, GIBCO BRL). Cultures were split with Trypsin-EDTA (GIBCO BRL) when they were subconfluent. The split ration was 1:5 to 1:10.

The cell lines CCL-34, CRL-6230 and CR1-6234 were purchased from ATCC, they were grown as described for cell line CC1-34 in Example 1.

To determine the level of EGFR expression, mRNA from all samples was prepared and polyA⁺ mRNA was isolated. These RNAs were separated by gel electrophoresis and Northern Blot analysis was performed. By using the expression level of the house-keeping gene GAPDH the EGFR expression levels of all cell lines except SH1b were determined.

EGF receptor expression of all used cell lines was examined on Northern Blots. For these Northern Blots fragment 3 (see Fig. 1) was used as a probe for the EGF receptor and GAPDH as the control.

Specifically, 1µg polyA⁺ RNA was separated on agarose gel, blotted onto nylon membranes and hybridized using a standard protocol. Hybridisation was always performed using the probe of interest - a probe selective for the EGFR - in combination with the house-keeping gene GAPDH. To detect the canine EGF-receptor the fragment 3 of the receptor was used. The same amount of both DNA probes was labeled with the same batch of ³²P-ATP and the same amount of incorporated cpm per ml was used for the hybridisation (4x10⁶ cpm/ml). The evaluation of the blots was done using a phosphoimager (Molecular Dynamics).

The cell line SLU-1 showed the highest expression of EGF receptor, SLU-2, SLU-4 and CCL-34 had lower, but detectable, expression. The last four cell lines, SLU-3, p114 (van Leeuwen et al., 1996), CRL-6230 and CRL-6234, did not express detectable levels of EGF receptor.

### b) Expression analysis using RT-PCR

From the mRNA of the cell lines SH1b, p114, CCL-34, SLU-1 and A431 (as a positive control) cDNA was made using a standard protocol. With these cDNAs PCR was performed using the primer pair F3 (SEQ ID NO:7) and R3 (SEQ ID NO:8).

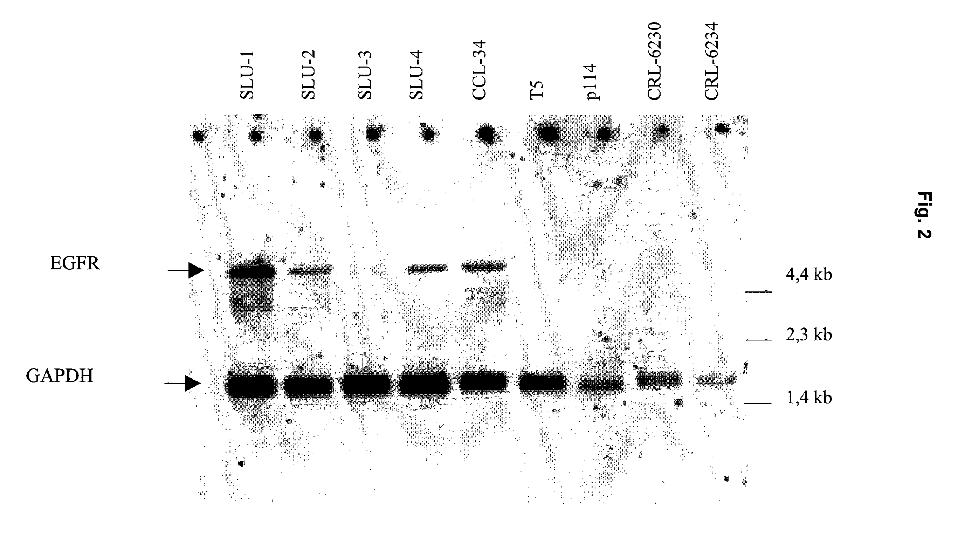

The PCR for was run with different numbers of cycles (12, 16, 20, 24, 28 cycles) at the following temperatures and times: 95°C (30 seconds), 59°C (30 seconds) and 72°C (1 minute). The PCR fragments were analysed at an agarose gel (the Northern Blot is shown in Fig. 2).

### c) ³H-Thymidin proliferation assay

The ability of BIBX1382 to inhibit the proliferation of cells in culture was tested with a ³H-Thymidin proliferation assays. This assay was performed with three tumor cell lines (SLU-1, SLU-4, SH1b) and a normal cell line (CRL-6234).

A pilot experiment with 500 to 12.000 cells per well in a 96-well plate in the presence of different concentrations of FCS (0% to 10%) was performed. After different proliferation times (24h, 48h, 72h and 96h), 10µl ³H-Thymidin (1:100 in PBS) were added, after incubation the plate was frozen, then thawed and the cells were harvested. The ³H-Thymidin incorporation rate was determined. For subsequent experiments the conditions (number of cells, FCS concentration) were chosen that showed the highest uptake of ³H-Thymidin. To determine a possible growth inhibitory effect of the inhibitor BIBX1382 on the cell lines, different concentrations (5nM-10000nM) of the inhibitor were used. Again ³H-Thymidin was added, after incubation the plate was frozen, then thawed and the cells were harvested. The ³H-Thymidin incorporation rate was determined in dependance of the concentration of inhibitor.

Cells of the cell line SLU-1 were inhibited in their proliferation rate by BIBX1382. The proliferation rate of SLU-4 cells was not affected by BIBX1382. For the cell line CRL-6234, BIBX1382 had toxic effects at high concentrations, at lower concentration CRL-6234 cells were not inhibited.

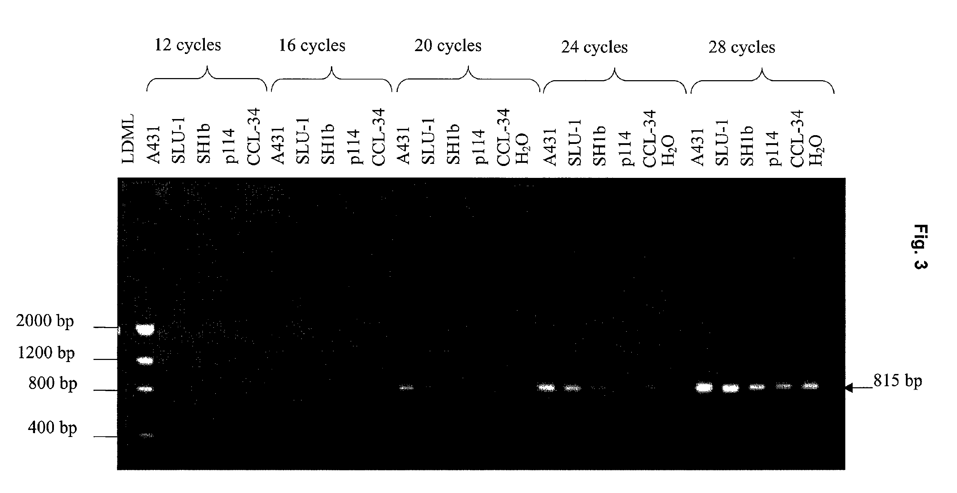

The ³H-Thymidin proliferation assay was also done with the tumor cell line SH1b. This cell line was inhibited in its proliferation time by BIBX1382 (Fig. 3).

### Example 3

Effect of the EGFR inhibitor BIBX1382 on tumor growth in nude mice

The cell line SLU-1 was cultured as described in Example 2. For use in tumor experiments, cells were trypsinised, washed and suspended in PBS + 5% FCS at 10⁷ cells/ml. 2x10⁶ SLU-1 cells were injected into 40 nude mice (5-10 week-old female Hsd:NMRI-nu/nu purchased from Harlan, The Netherlands for the *in vivo* experiment with BIBX1382.

When tumors were established and had reached diameters of approximately 5 mm as measured with a calliper, mice were randomly distributed into groups of 10 animals so that each tumor size was equally represented in each treatment group. The time span between cell injection and randomisation/start of treatment was sixteen days. Starting ten days after randomisation, mice were treated once daily (at 24 hour intervals) with various doses of the test compound. Administration was intragastral using a gavage needle (Infusionskanüle Olive A, Acufirm, No. 14 64 11 - 1). Therefore, the compound was suspended in 2.5% aqueous HP-beta-CD (hydroxy-propyl-beta-cyclodextrine)/1% aqueous Natrosol (hydroxyethylcellulose). Control mice received the vehicle only. The administration volume was 2.5 to 10 ml per kg mouse body weight. The solutions were proportioned and kept at -20° C.

The low dose group received 25 mg/kg/day (day 10-22) and 50 mg/kg/day (day 23-46) of BIBX1382; whereas the high dose group received 50 mg/kg/day (day 10-22), 70 mg/kg/day (day 23-30) and 100 mg/kg/day (day 31-46) of BIBX1382. The control group was treated with vehicle only.

Tumors were measured three times a week (Monday, Wednesday and Friday) with a calliper. Volumes [in mm³] were then calculated according to the formula (tumor volume = length * diameter² * π/6). To monitor side effects of treatment, mice were inspected daily for abnormalities and weighed three times (Monday, Wednesday and Friday) a week.

Tumor volumes were expressed as mean absolute tumor volumes [in mm³] and plotted over days of treatment.

The specific relative tumor volumes (T/C [%], i. e. the mean relative tumor volumes of groups of compound-treated tumors in % of the mean relative tumor volume of the vehicle-treated control tumors: T/C [%] = 100 * mean Vᵣₑₗ compound-treated / mean Vᵣₑₗ vehicle-treated) were determined on the day following the final day of treatment.

Experiments were usually terminated when vehicle control mice had to be killed because of large tumor sizes (1200 to 1700mm³).

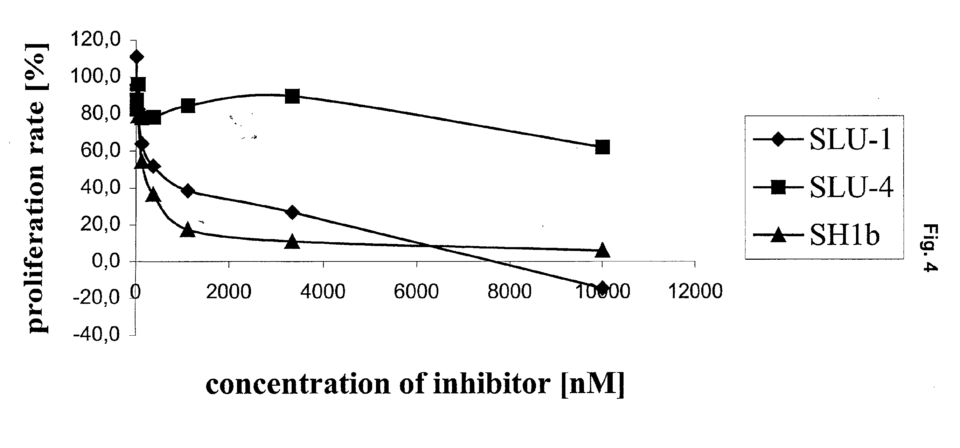

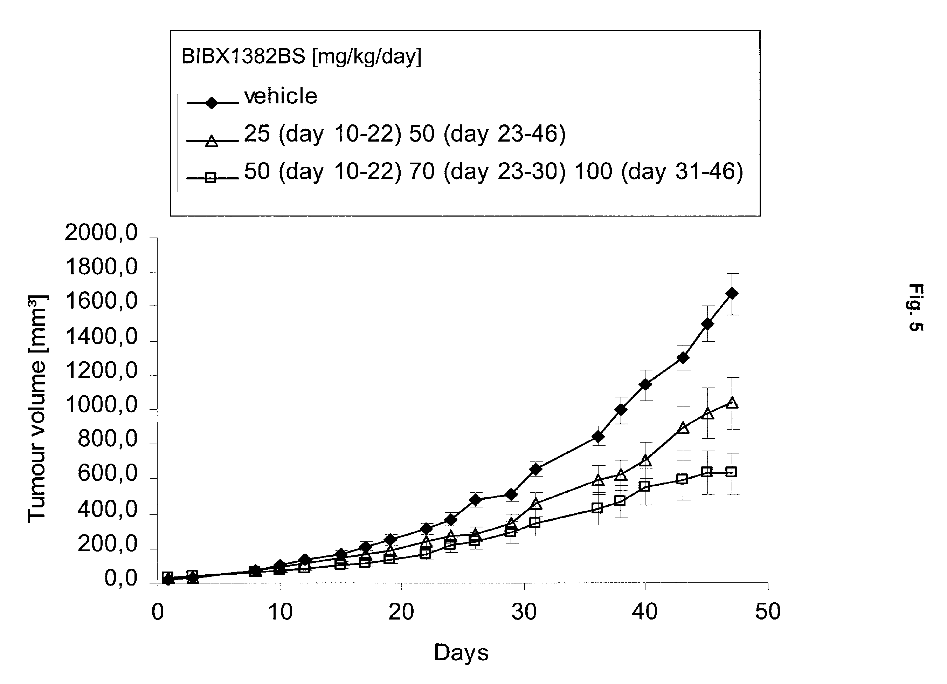

As shown in figure 4 and 5, BIBX1382 influenced SLU-1 tumor growth at all tested doses in the tumor inhibiting experiment. The volumes of all 10 tumors in the vehicle control mice increased (on average >60-fold over 7 weeks, albeit at different individual rates and with frequent intervals of growth interruption). The low dose group was treated with 25 mg/kg/day BIBX1382 from day 10 to day 22. During this time period 9 of 10 tumors showed a 8-fold increase in size, whereas the volume of one tumor did not change at all. From day 23 to day 46 this low dose group was treated with 50 mg/kg/day BIBX1382. All 10 tumors grew during this period showing a 3-6-fold increase in size. Looking at the whole period of treatment (day 10 - 46), the tumor size increased on average 35-fold.

The treatment of the high dose group started with a dose of 50 mg/kg/day from day 10 to day 22. During this period all ten tumors increased in size and showed at maximum a 5-6-fold increase. After increasing the dose to 70 mg/kg/day (day 23 - 30) the tumors grew by about 2-fold. From day 31 to day 46 the dose was raised to 100 mg/kg/day and the size of the tumors increased about 1.5-1.9-fold. Looking at the whole period of treatment again, the tumors increased in size on average 20-fold.

These responses of SLU-1 tumors to BIBX1382 are also reflected by T/C [%]-values (Table 5). After treatment of the low dose group with 25 mg/kg/day (day 10-22) the T/C- value was 78%. Raising the dose to 50 mg/kg/day (day 23-46) led to a decrease of the T/C-value to 62%. BIBX1382 treatment in the high dose group started with a dose of 50 mg/kg/day (day 10-22) leading to a T/C-value of 52%. Increasing the doses to 70 mg/kg/day (day 23-30) resulted in a T/C-value of 53%. After raising the dose to 100 mg/kg/day (day 31-46) the T/C-value decreased to 38%. Statistical analysis revealed that the T/C values of both dose groups (low and high) were significantly lower (standard T-test) compared to the vehicle control group.

The plasma levels of BIBX1382 determined after the last compound administration are shown in Table 6. These data demonstrated that plasma levels of approximately 4000 nmol/l at 2 hours after oral compound administration are able to reduce the tumor growth rate of SLU-1 as subcutaneous xenograft to a T/C value of 38%. With plasma levels of 3000 nmol/l at 2 hours after oral compound administration a reduction of the growth rate to a T/C value of 62% was achieved.

The results of the tumor inhibiting experiment are presented as plots of the mean absolute tumor volumes by group. T/C [%] along with the evaluations of tumor responses are shown in Table 5.

**Table 5**

| Response of SLU-1 tumors to BIBX1382 | | | | | |
|---|---|---|---|---|---|
| Experiment#¹ | Group | Daily oral dose [mg/kg]⁴ | Duration of treatment [days]² | T/C [%]³ | Tumor response |
| DMT05/00 | 1 | 25→50 | 13, 24 | 62 | partial growth suppression |
| | 2 | 50→70→100 | 13, 8, 16 | 38 | growth suppression |
| | control | vehicle | 37 | 100 | - |

| | | | | | |
|---|---|---|---|---|---|
| ^{*1*} *Start of treatment was 16 days after injection of 2x10*^{*6*} *SLU-1 cells in experiment DMT05*/*00.* | | | | | |
| ^{*2*} *The first number indicates the length of the treatment period for the first dose followed by the figures indicating the length of treatment at the following doses.* | | | | | |
| ^{*3*} *Determined on the day following the final day of treatment of the vehicle control group (day 47).* | | | | | |
| ⁴ Doses were raised to obtain a maximum difference between the BIBX1382-treated and vehicle-treated mice. | | | | | |

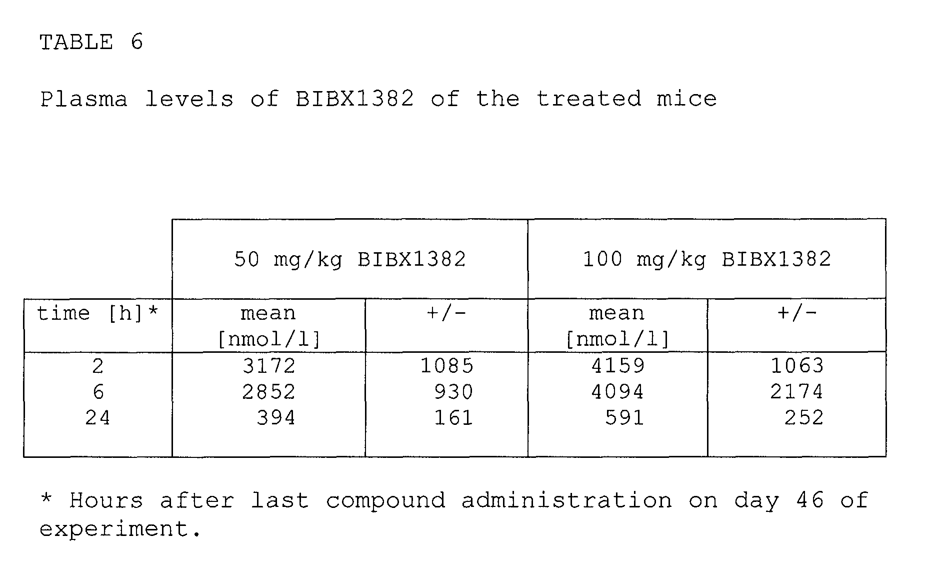

### Example 4

### Histological analysis of EGF receptor expression in canine tumors

### a) Histological examination of paraffin sections of canine mamma-adenocarcinomas with anti-EGFR antibodies

Immunohistochemical stainings were performed with paraffin sections of canine mamma-adenocarcinomas in order to determine whether the canine EGF receptor can be detected with a human EGFR-antibody.

Specific staining on the paraffin sections of canine mamma-adenocarcinomas was done with a human EGFR antibody (Santa Cruz cat# sc-03). The sections were stained using a standard protocol for the streptavidin-biotin-complex method.

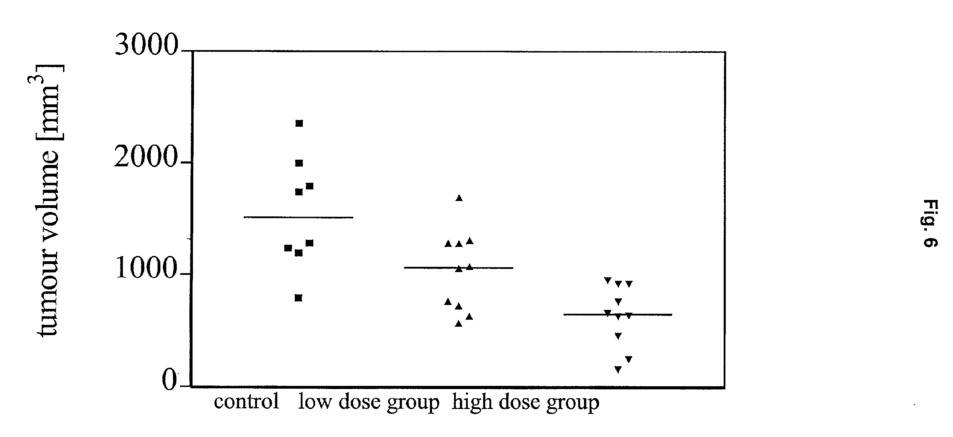

The paraffin sections of 22 different tumors were stained with this antibody. In two cases the sections could not be analysed. All other cases showed a specific staining of the EGF receptor. The levels of EGF receptor expression of the tumor cells are comparable to those of, for example, normal skin cells. This means that the EGF receptor is not overexpressed in these tumor samples, but it is expressed in 91% of the tested samples (Figure 6).

### b) Detection of EGFR on the cellular level

The cytospin analysis was performed with the cell lines SLU-1, SLU-2, SLU-3, SLU-4, T5, p114, SH1b and CCL-34. 10.000 cells were centrifuged (10 minutes, 800 rpm) onto a slide and fixed with paraformaldehyd. The staining was done using the streptavidin-biotin-complex method used for the staining of the paraffin-sections.

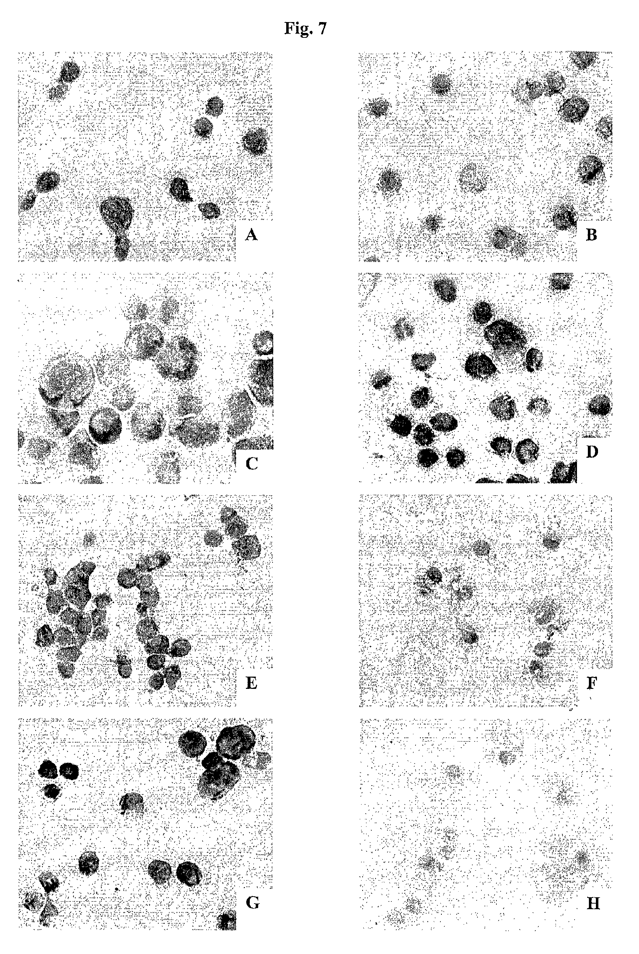

The cell lines SLU-1, CCL-34 and SLU-4 showed a high expression of the EGF-receptor, SH1b, SLU-2 and SLU-3 had lower expression. On the cytospin of the cell lines p114 only a few cells were stained, on the slide of T5 no cell was stained (Figure 7). These data show that this antibody can be used for the analysis of canine tumor biopsies.

### Example 5

### Cloning of the canine HER2-receptor

For these experiments, the same methods are applied as for cloning of the canine EGFR described in Example 1.

For cloning the canine HER2 receptor, following a strategy similar to that described for the canine EGFR in Example 1, the major part of the intracellular domain, the whole transmembrane and a small part of the extracellular domain are used as templates (Coussens et al; 1985). Three PCR primer pairs (SEQ ID NO:9 and SEQ ID NO:10; SEQ ID NO:11 and SEQ ID NO:12; SEQ ID NO:13 and SEQ ID NO:14) are used to encompass these defined area of the human sequence.

The positions of the primers in the human HER2-receptor sequence are the following:
SEQ ID NO: 9: 1800-1819, SEQ ID NO: 10: 3140-3124;
SEQ ID NO: 11 2807-2828, SEQ ID NO: 12 3783-3764;
SEQ ID NO: 13 3458-3481, SEQ ID NO: 14 4481-4458.
Messenger RNA from normal canine cell lines (e.g. CRL-6230, CRL-6234 and CCL-34) is isolated and transcribed into cDNA. These cDNA samples are used in different PCR reactions with the three primer pairs defined above. The resulting three PCR-fragments from the different primer pairs are subcloned into the TA vector system and sequenced. Cloning of the HER2 receptor can be completed using the SMART-PCR technology, as described in Example 1.

### Example 6

### Galenic formulations of the EGFR inhibitor BIBX1382

### a) Coated tablets containing 75 mg of BIBX1382

| 1 tablet core contains: | |
|---|---|
| BIBX1382 | 75.0 mg |
| calcium phosphate | 93.0 mg |
| corn starch | 35.5 mg |
| polyvinylpyrrolidone | 10.0 mg |
| hydroxypropylmethylcellulose | 15.0 mg |
| magnesium stearate | 1.5 mg |
| | 230.0 mg |

### Preparation:

BIBX1382 is mixed with calcium phosphate, corn starch, polyvinylpyrrolidone, hydroxypropylmethylcellulose and half the specified amount of magnesium stearate. Blanks 13 mm in diameter are produced in a tablet-making machine and these are then rubbed through a screen with a mesh size of 1.5 mm using a suitable machine and mixed with the rest of the magnesium stearate. This granulate is compressed in a tablet-making machine to form tablets of the desired shape.
- Weight of core:: 230 mg
- Diameter:: 9 mm, convex

The tablet cores thus produced are coated with a film consisting essentially of hydroxypropylmethylcellulose. The finished film-coated tablets are polished with beeswax.
- Weight of coated tablet:: 245 mg.

### b) Tablets containing 100 mg of BIBX1382

### Composition:

| 1 tablet contains: | |
|---|---|
| BIBX1382 | 100.0 mg |
| lactose | 80.0 mg |
| corn starch | 34.0 mg |
| polyvinylpyrrolidone | 4.0 mg |
| magnesium stearate | 2.0 mg |
| | 220.0 mg |

### Preparation:

BIBX1382, lactose and starch are mixed together and uniformly moistened with an aqueous solution of the polyvinylpyrrolidone. After the moist composition has been screened (2.0 mm mesh size) and dried in a rack-type drier at 50°C it is screened again (1.5 mm mesh size) and the lubricant is added. The finished mixture is compressed to form tablets.
- Weight of tablet:: 220 mg
- Diameter:: 10 mm, biplanar, facetted on both sides and notched on side.

### c) Tablets containing 150 mg of BIBX1382

### Composition:

| 1 tablet contains: | |
|---|---|
| BIBX1382 | 150.0 mg |
| powdered lactose | 89.0 mg |
| corn starch | 40.0 mg |
| colloidal silica | 10.0 mg |
| polyvinylpyrrolidone | 10.0 mg |
| magnesium stearate | 1.0 mg |
| | 300.0 mg |

### Preparation:

BIBX1382 mixed with lactose, corn starch and silica is moistened with a 20% aqueous polyvinylpyrrolidone solution and passed through a screen with a mesh size of 1.5 mm. The granules, dried at 45°C, are passed through the same screen again and mixed with the specified amount of magnesium stearate. Tablets are pressed from the mixture.
- Weight of tablet:: 300 mg
- Diameter:: 10 mm, flat

### d) Hard gelatine capsules containing 150 mg of BIBX1382

| 1 capsule contains: | | |
|---|---|---|
| BIBX1382 | | 150.0 mg |
| corn starch (dried) | approx. | 180.0 mg |
| lactose (powdered) | approx. | 87.0 mg |
| magnesium stearate | | 3.0 mg |
| | approx. | 420.0 mg |

### Preparation:

BIBX1382 is mixed with the excipients, passed through a screen with a mesh size of 0.75 mm and homogeneously mixed using a suitable apparatus. The finished mixture is packed into size 1 hard gelatine capsules.
- Capsule filling:: approx. 320 mg
- Capsule shell:: size 1 hard gelatine capsule.

### References

Baselga J., Mendelsohn J., 1997, *Type I receptor tyrosine kinases as targets for therapy in breast cancer.* J Mammary Gland Biol Neoplasia, Apr;2(2):165-74
Baselga J., 2000, *Current and planned clinical trials with trastuzumab (Herceptin).* Semin Oncol, Oct;27 (5 Suppl 9):27-32
Bonnett B.N., Egenvall A., Olson P., Hedhammar A., 1997, *Mortality in insured Swedish dogs: rates and causes of death in various breeds.* The Veterinary Report, July 12
Coussens L., Yang-Feng T.L., Liao Y.C., Chen E., Gray A., McGrath J., Seeburg P.H., Libermann T.A., Schlessinger J., Francke U., Levinson A., Ullrich A., 1985, *Tyrosine Kinase Receptor with Extensive Homology to EGF Receptor Shares Chromosomal Location with neu Oncogene.* Science Vol. 230, 6 December, 1132-1139
Davies D.E. and Chamberlin S.G., 1996, Biochem. Pharmacol., 51, 1101-1110
Donnay I., Rauis J., Wouters-Ballman P., Devleeschouwer N., Leclercq G., Verstegen J.P., 1993, *Receptors for oestrogen, progesterone and epidermal growth factor in normal and tumorous canine mammary tissues.* J. Reprod. Fert., Suppl. 47, 501-512
Earp H.S., Dawson T.L., Li X., Yu H., 1995, *Heterodimerization and functional interaction between EGF receptor family members: a new signaling paradigm with implications for breast cancer research.* Breast Cancer Res Treat Jul;35(1):115-32
Ferrer C., Anastasi A.M., Di Massimo A.M., Bullo A., Di Loreto M., Raucci G., Pacilli A., Rotondaro L., Mauro S., Mele A., De Santis R., 1996, *Expression and characterization of a mouse*/*human chimeric antibody specific for EGF receptor.* J Biotechnol Nov 29; 52(1):51-60
Hellmén E., 1992, *Characterization of Four In Vitro Established Canine Mammary Carcinoma and One Atypical Benign Mixed Tumor Cell Lines.* In Vitro Cell. Dev. Biol. 28A: 309-319, May
Hellmén E., 1993, *Canine Mammary Tumor Cell Lines Established In Vitro.* J. Reprod. Fert. Supll. 47, 489-499
Hellmén E., 1992, *Characterization of Four In Vitro Established Canine Mammary Carcinoma and One Atypical Benign Mixed Tumor Cell Lines.* In Vitro Cell. Dev. Biol. 28A: 309-319
Hellmén E., Moller M., Blankenstein M.A., Andersson L., Westermark B., 2000, *Expression of Different Phenotypes in Cell Lines from Canine Mammary Spindle-Cell Tumors and Osteosarcomas indicating A Pluripotent Mammary Stem Cell Origin.* Breast Cancer Research and Treatment 61: 197-210
Klohs et al., 1997, Curr.Opin.Oncol. 9,562
Levitzky, A., 1994, *Signal tranduction therapy.* Eur. J. Biochem., 226:1-13
Levitzky, 1999, Pharmacol Ther. 82, 231
Luetteke N.C., Phillips H.K., Qiu T.H., Copeland N.G., Earp H.S., Jenkins N.A., Lee D.C., 1994, *The Mouse Waved-2 Phenotype Results from a Point Mutation in the EGFR Receptor Tyrosine Kinase.* Genes & Development 8, 399-413
Mc Mahon et al., 1998, Current Opinion in Drug Discovery & Development 1 (2): 131-146
Modjtahedi, H. and Dean, C., 1994, *The receptor for EGF and its ligands: expression, prognostic value and target for therapy in cancer.* Int.J.Oncology 4: 277-96
Nerurkar V.R., Seshadri R., Mulherkar R., Ishwad C.S., Lalitha V.S., Naik S.N., 1987, *Receptors for Epidermal Growth Factor and Estradiol in Canine Mammary Tumors.* Int. J. Cancer: 40, 230-232
Peth L.A., Harris J., Raymond V.W., Blasband A.J., Lee D.C., Earp H.S., 1990, *A Truncated, Secreted Form of the Epidermal Growth Factor Receptor Is Encoded By an Alternatively Spliced Transcript in Normal Rat Tissue.* Mol. Cell. Biol. 10, 2973-2982
Strawn and Shawver, 1998 Expert Opinion On Investigational Drugs, 7 (4), 553 -573)
Ullrich A., Coussens L., Hayflick J.S., Dull T.J., Gray A., Tam A.W., Lee J., Yarden Y., Libermann T.A., Schlessinger J., Downward J., Mayes E.L.V., Whittle N., Waterfield M.D., Seeburg P.H., 1984, *Human Epidermal Growth Factor Receptor cDNA Sequenzce and Abberant Expression of the Amplified Gene in A531 Epidermoid Carcinoma Cells.* Nature Vol. 309, 31 May
van der Burg B., van Selm-Miltenburg A., van Maurik P., Rutteman G.R., Misdorp W., de Laat S.W., van Zoelen E., 1989, *Isolation of Autonomously Growing Dog Mammary Tumor Cell Lines Cultured in Medium Supplemented With Serum Treated To Inactivate Growth Factors.* Journal of the National Cancer Institute, Vol. 81, No. 20, October 18
van Leeuwen I.S., Hellmén E., Cornelisse C.J., van den Burgh B., Rutteman G.R., 1996, *P53 Mutations in Mammary Tumor Cell Lines and Corresponding Tumor Tissues in the Dog.* Anticancer Research 16: 3737-3744
Walter J.H., Gutberlet K., Schwegler K., Rudolph R., 1997, *Vorkommen und Häufigkeit caniner Neoplasien im Sektionsgut.* Kleintierpraxis, 42. Jahrgang, 273-284
Walter J.H., Schwegler K., 1992, *Untersuchungen zur Häufigkeit von Neoplasien bei sezierten Hunden in Berlin (West).* J. Vet. Med. A 39, 328-341
Woodburn, 1999; Pharmacol.Ther.82:241

## Claims

1. Pharmaceutical composition for the treatment of mammary tumors of a non-human mammal, which is associated with an aberrant activity of one or more tyrosine kinase receptors, wherein said pharmaceutical composition contains, as the active ingredient, one or more substances that inhibit the aberrant activity of the tyrosine kinase receptor(s) of said animal.

2. The pharmaceutical composition of claim 1 for the treatment of canine mammary tumors.

3. The pharmaceutical composition of claim 1 or 2, wherein the active ingredient is an inhibitor of a class I tyrosine kinase receptor.

4. The pharmaceutical composition of claim 3, wherein the active ingredient is a compound that blocks the ATP binding site in the kinase domain of the tyrosine kinase receptor.

5. The pharmaceutical composition of claim 3 or 4, wherein the active ingredient is an inhibitor of the Epidermal Growth Factor Receptor (EGFR).

6. The pharmaceutical composition of claim 3, wherein the active ingredient is a compound that inhibits the aberrant activity of the corresponding human receptor.

7. The pharmaceutical composition of any one of claims 3 to 6, wherein the inhibitor is a pyrimido-pyrimidine.

8. The pharmaceutical composition of claim 7, wherein the inhibitor is 4-((3-chloro-4-fluorophenyl)amino)-6-(1-methyl-4-piperidinyl-amino)-pyrimido(5,4d)pyrimidine).

9. The pharmaceutical composition of claim 3, wherein the active ingredient is an inhibitor of HER2.

10. The pharmaceutical composition of claim 3, wherein the active ingredient is a combination of an EGFR inhibitor and a HER2 inhibitor.

11. The pharmaceutical composition of claim 9, wherein the EGFR inhibitor is 4-((3-chloro-4-fluorophenyl)amino)-6-(1-methyl-4-piperidinyl-amino)-pyrimido(5,4d)pyrimidine).

12. Use of a compound that has the ability to inhibit the aberrant expression of a class I tyrosine kinase receptor in humans for the preparation of a pharmaceutical composition for the treatment of canine mammary tumors.

13. The use of claim 12, wherein the compound is an EGFR inhibitor.

14. The use of claim 13, wherein the EGFR inhibitor is 4-((3-chloro-4-fluoro-phenyl)amino)-6-(1-methyl-4-piperidinyl-amino)-pyrimido(5,4d)pyrimidine).

15. The use of a compound that has the ability to inhibit the aberrant expression of a class I tyrosine kinase receptor in humans for the preparation of a pharmaceutical composition for the treatment of mammary tumors in a non-human mammal whose receptor has an identity of 100% with the corresponding human receptor on the amino acid level.
